# EUROPEAN PATENT SPECIFICATION

(11) **EP 3 323 429 B1**
(45) Date of publication and mention of the grant of the patent: **12.02.2025**
(21) Application number: 16824498.6
(22) Date of filing: 13.07.2016
(51) Int. Cl.: A61K 47/36, A61K 8/86, A61K 38/22, A61K 38/28, A61P 3/10, A61Q 19/00

(54) **DRUG SUSTAINED-RELEASE CARRIER AND METHOD FOR PRODUCING SAME**
TRÄGER MIT VERZÖGERTER ARZNEIMITTELABGABE UND VERFAHREN ZUR HERSTELLUNG DAVON
SUPPORT À LIBÉRATION PROLONGÉE DE MÉDICAMENT ET SON PROCÉDÉ DE PRODUCTION

(30) Priority: 13.07.2015 JP 2015139806
(43) Date of publication of application: 23.05.2018
(73) Proprietor: Cosmed Pharmaceutical Co., Ltd., Kyoto-shi, Kyoto 601-8014 (JP); National University Corporation Kobe University, Kobe-city, Hyogo 657-8501 (JP)
(72) Inventor: QUAN, Ying-shu, Kyoto-shi Kyoto 601-8014 (JP); OOYA, Tooru, Kobe-shi Hyogo 657-0013 (JP); JIANG, Rongrong, Kyoto-shi Kyoto 601-8014 (JP); KAMIYAMA, Fumio, Kyoto-shi Kyoto 601-8014 (JP)
(74) Representative: Ter Meer Steinmeister & Partner
(86) International application number: PCT/JP2016/070704
(87) International publication number: WO 2017/010518

(56) References cited:
- EP-A1- 2 213 315
- JP-A- 2013 507 490
- US-A1- 2010 316 682
- MORIYAMA,K. ET AL.: "Hyaluronic acid grafted with poly(ethylene glycol) as a novel peptide formulation", JOURNAL OF CONTROLLED RELEASE, vol. 59, 1999, pages 77 - 86, XP004166217
- YADAV,A.K. ET AL.: "Development and characterization of hyaluronic acid-anchored PLGA nanoparticulate carriers of doxorubicin", NANOMEDICINE: NANOTECHNOLOGY, BIOLOGY, AND MEDICINE, vol. 3, 2007, pages 246 - 257, XP022394224
- D'ESTE,M. ET AL.: "A systematic analysis of DMTMM vs EDC/NHS for ligation of amines to Hyaluronan in water", CARBOHYDRATE POLYMERS, vol. 108, 2014, pages 239 - 246, XP055203474
- LOEBEL,C. ET AL.: "Precise tailoring of tyramine- based hyaluronan hydrogel properties using DMTMM conjugation", CARBOHYDRATE POLYMERS, vol. 115, 22 January 2015 (2015-01-22), pages 325 - 333, XP029019642

## Description

### Technical Field

The present invention relates to development of a novel drug-sustained-release carrier using a novel drug delivery technique.

### Background Art

A combination of a particular salt solution and an aqueous polymer solution or a combination of different aqueous polymer solutions exhibits liquid-liquid phase separation when certain conditions such as concentration and temperature are met. The separated two liquid phase systems are called as an aqueous two-phase system. When a substrate such as a protein is added to this aqueous two-phase system, the substrate is unevenly distributed because of difference in the substrate diffusion between the two phases. So far, many combinations of aqueous polymers and salts constituting the aqueous two-phase system have been discovered, and they have been applied to the technology for extracting protein medicines.

According to physicochemical study of an aqueous two-phase system between aqueous polymer solutions, a cause of phase separation is considered to be repulsion between monomer units constituting the polymers. In the environment in which heterogeneous polymers coexist, a mixing entropy is extremely small because of chain combination as compared with the case where monomer units independently exist. Thus, it is considered that phases are separated from each other by an effect of repulsive force acting between monomer units. The aqueous two-phase system is widely used as a means for separation and purification.

Advantages of the aqueous two-phase distribution method include the following four points.
(1) Adsorption of biological substances and the accompanying structural destruction/denaturation and the like can be avoided because of using no solid phase, and the coexistence of the hydrophilic polymers provides stabilization, allowing operation at normal temperature.
(2) Since the difference in the interfacial tension between the two phases is slight, a fine droplet can be suspended in a relatively stable state when the two phases are mixed, and distribution equilibrium can be quickly and easily achieved.
(3) When a hydrophilic group is introduced to a target substance, specificity of separation can be improved.
(4) Since its distribution coefficient is insusceptible to the total volume of the system, the volume ratio between the two phases and the concentrations of the subjects to be separated, scale up can be easily carried out based on experimental results on the test tube scale.

On the other hand, sustained release of not only protein medicines but also low-molecular weight drugs is a great challenge in the fields of pharmacology and medicine, and there is not yet sufficient solution. The carrier for sustained release is highly required in various dosage forms such as an injection, an internal medicine, an implant preparation, a microneedle preparation and a percutaneously absorbable preparation, and realization thereof is desired.

There have already been some reports on a sustained release drug delivery system (hereinafter abbreviated as "DDS") using the aqueous two-phase distribution method. For example, sustained-release administration of a proteinaceous medicine using a polyethylene glycol aqueous solution as a continuation phase and a crosslinkable dextran polymer aqueous solution as a dispersion phase (Patent Document 1), and sustained-release administration of a protein (cytokine) using an emulsion formed by using a water-soluble polysaccharide aqueous solution as a continuation phase and a polyethylene glycol diacrylate aqueous solution as a dispersion phase (Patent Document 2) have already been reported. Hereinafter, polyethylene glycol will be abbreviated as PEG. Patent Document 3 provides a biodegradable hyaluronic acid derivative including at least one modified hyaluronic acid repeating unit represented by the formula (HA)-[O(C=O)NH-M]p, wherein HA is a unit including N-acetyl-D-glucosamine and D-glucuronic acid, M is a modifying moiety containing a C2-16 hydrocarbyl group, and p is an integer of 1 to 4. Patent Document 4 relates to hydrogels endowed with antibacterial properties, to be used for injection in damaged bones or in the production of antibacterial coatings of prostheses for implant in the human or animal body.

However, when a proteinaceous medicine or the like was administered into a body in a sustained manner, even if it was attempted to utilize the phase separation between a polyethylene glycol aqueous solution and a crosslinkable dextran polymer aqueous solution, the size of the dispersion phase, the equilibrium distribution ratio to two phases and the like were hardly controlled, and stable sustained release of a drug was difficult.

### Prior Art Documents

### Patent Documents

Patent Document 1: Japanese Translation of PCT International Application Publication No. JP-T- 2001-504828
Patent Document 2: Japanese Translation of PCT International Application Publication No. JP-T- 2006-517523
Patent Document 3: US 2010 316682 A1
Patent Document 4: EP 2 213 315 A1

### Summary of Invention

### Problem to be solved

An object of the present invention is to provide a novel DDS carrier based on a principle of an aqueous two-phase system. Although an aqueous two-phase distribution phenomenon aimed at application of protein medicine to sustained release preparations is currently studied, usefulness of a PEG-grafted polymer as a DDS carrier has not yet been clarified. Furthermore, its production method has not yet been established, and distribution condition control of proteins distributed to a PEG phase, release control and the like have not yet been studied. The present invention provides a novel production method for a compound prepared by grafting a one-terminal-aminated PEG (PEG-NH₂) having a repeating unit structure of PEG in a polymer. Also, it aims to apply the compound to a DDS for sustained release of a protein drug.

### Solution to Problem

The DDS carrier according to the present invention made to solve the above-described problems is a PEG-carboxy group-containing polysaccharide graft, wherein a carboxy group and PEG are grafted through an ester bond, wherein the carboxy group-containing polysaccharide is hyaluronic acid, and wherein a weight ratio of PEG and hyaluronic acid is 1 : 20 to 1 : 0.1 as defined in claim 1. Further aspects and embodiments of the invention are defined in claims 2 to 10.

Herein, the PEG-carboxy group-containing polymer (polysaccharide) graft means a compound prepared by grafting PEG together with a carboxy group-containing polymer (polysaccharide) as a main chain. Suitably, it means a compound obtained by a process that an amino group is introduced into PEG, a carboxy group is introduced into a polymer (polysaccharide) or alternatively a carboxy group-containing polymer is used to esterify the polymer by a base or an acid catalyst in an anhydrous environment, and thereby the carboxy group in the polymer (polysaccharide) and the PEG-terminal hydroxy group are condensed. The structure of the PEG-carboxy group-containing polymer graft can be confirmed by ¹H-NMR spectrum.

When PEG and the carboxy group-containing polymer are dissolved in water, the aqueous two-phase system is formed. When a proteinaceous medicine is dissolved therein, quite a lot of proteinaceous medicine is distributed to the PEG phase. However, even if an attempt to administer the proteinaceous medicine condensed into the PEG phase by means of this aqueous two-phase system to a body is made, PEG diffuses in body fluid (blood, intercellular fluid) and all proteinaceous medicine is immediately released. Hence the proteinaceous medicine could not be produced as a sustained release preparation.

Thus, PEG is grafted into a carboxy group-containing polymer to considerably increase the whole molecular weight and it is rendered a swollen body rather than a dissolved product in water or body fluid, so that PEG can be prevented from diffusing in body fluid. By using this system, sustained release of the proteinic medicine becomes possible. That is, according to the present invention, the principle that the proteinaceous medicine is condensed into the PEG phase in the aqueous two-phase system is utilized, and in order to allow the medicine to be used as an actual DDS preparation, PEG is grafted into the carboxy group-containing polymer to prevent PEG from diffusing in the body fluid.

The molecular weight of the carboxy group-containing water-soluble polymer is preferably in the range of about 5×10⁴ to 5×10⁶ dalton. If the molecular weight is within this range, different carboxy group-containing water-soluble polymers may be mixed, or the same carboxy group-containing water-soluble polymers having different molecular weights may be mixed for use. Furthermore, a polymer having a molecular weight within this range and a polymer having a molecular weight lower than this range may be mixed for use.

As the carboxy group-containing polysaccharide, a hyaluronic acid (hereinafter abbreviated as "HA") is used. The hyaluronic acid may be a metal salt such as a sodium salt or a potassium salt. Hereinafter, the PEG-carboxy group-containing hyaluronic acid graft is abbreviated as "PEG-graft-HA".

Many methods for chemically bonding the carboxy group-containing polysaccharide and PEG by condensation reaction have been proposed. It was found that grafting of PEG by esterification of the hyaluronic acid in a nonaqueous state was an extremely effective grafting method. In this case, the PEG-carboxy group-containing polysaccharide graft can be produced by condensing PEG and the carboxy group-containing polysaccharide in coexistence with an acid. The bond between the carboxy group-containing polysaccharide (hyaluronic acid) and PEG is characteristically an ester bond. When hyaluronic acid is esterified in a nonaqueous state, it can be esterified in the presence of an acid such as sulfuric acid and perchloric acid as a catalyst. Although THF, chloroform, DMA or the like may be used as the nonaqueous solvent, grafting reaction may be carried out by suspending hyaluronic acid in PEG in the presence of an acid with using no solvent.

Although dicyclohexylcarbodiimide (DCC), ethyldimethylaminopropyl carbodiimide (EDC) or the like can also be used as the condensing agent for the condensation reaction, triazine-based compounds represented by the following chemical formula (1) are often used. Wherein R¹ and R² represent short-chain (1 to 6 carbon atoms) alkyl groups, and X represents halogen atom. When R¹ and R² are methyl groups and X is chlorine, the compound is 4-(4,6-dimethoxy-1,3,5-triazine-2-yl)-4-methylmorpholinium chloride (DMTMM), which is the most frequently used condensing agent.

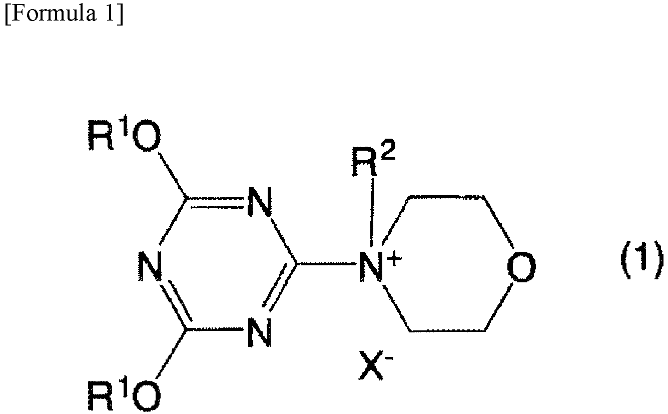

Hyaluronic acid is a type of glycosaminoglycan which is a long-chain polysaccharide. Glucuronic acid and acetylglucosamine are alternately bound through glycosidic bond of β-1,4 and β-1,3. While all of bulky substituents such as a hydroxy group, a carboxy group and an anomeric carbon binding to adjacent sugars are equatorial with steric advantage, all small hydrogen atoms form structures occupied by sterically more disadvantageous axial conformation. A substituent at an equatorial position forms a hydrophilic surface with strong polarity, hydrogen atom at an axial position is nonpolar and forms a relatively hydrophobic surface. As a result, the whole skeleton of the hyaluronic acid molecules in the physiological solution forms a ribbon-like and random coil structure and takes a very large volume.

The hyaluronic acid aqueous solution is colorless, transparent and gelatinous, and has a high viscosity. In the human body, it is contained in joint synovia and joint cartilage, and acts to assist functions of joints by a lubricating effect of smoothing motion between bones and a buffer effect of cushioning. Besides, it exists in a vitreous body of an eyeball and keeps its shape, and also acts to prevent invasion of bacteria. Also it acts to keep moisture and maintain a fresh springy feeling in a skin.

PEG is a polymer compound in which ethylene glycol is polymerized. Also it has been industrially used for a long time, and is widely used for applications of a nonionic surfactant, a lubricant, an intermediate for urethane synthesis, an adhesive, a cosmetic and the like. Furthermore, since PEG is nontoxic to human bodies, it is put into practical use as a pharmaceutical additive under the name "macrogol". In addition, since PEG is not captured by phagocytes of the cellular endothelial tissues typified by liver and spleen and provides properties of circulating and staying in blood for a long period (stealth property), PEGylation has been performed, in which PEG is added to a protein medicine or a liposome. Although the molecular weight of PEG is not particularly restricted in the present invention, the molecular weight is preferably 1,000 to 1,000,000. In addition, the structure of PEG is not particularly limited as long as the molecular weight is within this range.

The PEG-carboxy group-containing polysaccharide graft of the present invention can be used as a DDS carrier for proteinaceous medicines and low-molecular weight drugs. In particular, it is a compound which has both hydrophilic groups and hydrophobic groups in its molecule and can be suitably used for a compound having a high affinity with PEG.

In the PEG-carboxy group-containing polysaccharide graft, a weight ratio of PEG and the carboxy group-containing polysaccharide is 1 : 20 to 1 : 0.1 (weight ratio). If an amount of PEG to be grafted into the carboxy group-containing polysaccharide is too small, the affinity effect of PEG for proteinaceous medicines or low-molecular weight drugs is hardly expressed. In addition, a graft substance in which the amount of PEG to be grafted into the carboxy group-containing polysaccharide is too large is difficult to produce.

The molecular weight of the PEG-carboxy group-containing polysaccharide graft is not particularly restricted in the present invention and can be selected within a suitable range depending on the purpose, but in a case of a pharmaceutical DDS carrier, the molecular weight is preferably 200 to 20,000, and it may be a blend of PEGs having different molecular weights.

Drugs to which the present invention can be applied include the followings.

The proteinaceous medicine includes, for example, insulin, exendin-4, calcitonin, adrenocorticotropic hormone, parathyroid hormone (PTH), hPTH (1→34), secretin, oxytocin, angiotensin, β-endorphin, glucagon, vasopressin, somatostatin, gastrin, luteinizing hormone-releasing hormone, enkephalin, neurotensin, atrial natriuretic peptide, growth hormone, growth hormone-releasing hormone, bradykinin, substance P, dynorphin, thyroid-stimulating hormone, prolactin, interferon, interleukin, G-CSF, glutathione peroxidase, superoxide dismutase, desmopressin, somatomedin, endothelin, salts thereof, and the like. An antigen protein or a virus fragment includes influenza antigen, tetanus antigen, diphtheria antigen, HBs surface antigen, HBe antigen and the like.

The low-molecular weight drug is not particularly limited as long as it is a raw material for drugs and cosmetics which have been conventionally used. For example, it includes antipyretic analgesic, steroidal anti-inflammatory agent, vasodilator, antiarrhythmic agent, hypotensive agent, local anesthetic, hormonal agent, antihistamine, general anesthetic, soporific analgesic, antiepileptic agent, psychotropic agent, skeletal muscle relaxant, autonomic agent, antiparkinson agent, diuretic, vasoconstrictor, respiratory stimulant, narcotic and the like.

The antipyretic analgesic includes, for example, ibuprofen, flurbiprofen, ketoprofen and the like. The steroidal anti-inflammatory agent includes, for example, hydrocortisone, triamcinolone, prednisolone and the like. The vasodilator includes, for example, diltiazem hydrochloride, isosorbide nitrate and the like. The antiarrhythmic agent includes, for example, procainamide hydrochloride, mexiletine hydrochloride and the like.

The hypotensive agent includes, for example, clonidine hydrochloride, bunitrolol hydrochloride, captopril and the like. The local anesthetic includes, for example, tetracaine hydrochloride, propitocaine hydrochloride and the like. Examples of the hormonal agent include propylthiouracil, estradiol, estriol, progesterone and the like. The antihistamine includes, for example, diphenhydramine hydrochloride, chlorpheniramine maleate and the like.

The general anesthetic includes, for example, pentobarbital sodium and the like. The soporific analgesic includes, for example, amobarbital, phenobarbital and the like. The antiepileptic agent includes, for example, phenytoin sodium and the like. The psychotropic agent includes, for example, chlorpromazine hydrochloride, imipramine hydrochloride, chlordiazepoxide, diazepam and the like. The skeletal muscle relaxant includes, for example, suxamethonium hydrochloride, eperisone hydrochloride and the like.

The autonomic agent includes, for example, neostigmine bromide, bethanechol chloride and the like. The antiparkinson agent includes, for example, amantadine hydrochloride and the like. The diuretic includes, for example, hydroflumethiazide, isosorbide, furosemide and the like. The vasoconstrictor includes phenylephrine hydrochloride and the like. The respiratory stimulant includes, for example, lobeline hydrochloride, dimorpholamine, naloxone hydrochloride and the like. The narcotic includes, for example, morphine hydrochloride, cocaine hydrochloride, pethidine hydrochloride and the like.

The raw material for the cosmetic includes, for example: a whitening ingredient such as ascorbyl palmitate, kojic acid, rucinol, tranexamic acid, oiliness liquorice extract and vitamin A derivative; an anti-wrinkle ingredient such as retinol, retinoic acid, retinol acetate and retinol palmitate; a blood circulation-promoting ingredient such as tocopherol acetate, capsin and nonylic acid vanillylamide; a diet ingredient such as raspberry ketone, evening primrose extract and seaweed extract; an antimicrobial ingredient such as isopropyl methylphenol, photosensitizer and zinc oxide; a vitamin such as vitamin D2, vitamin D3 and vitamin K; and the like.

The PEG-graft-HA can be applied to various dosage forms for sustained release of drugs. For example, applications in e.g. an injection, an internal medicine, an implant preparation, a percutaneous absorbent preparation, a microneedle preparation; and a cosmetic such as cosmetic lotion, cosmetic emulsion, cosmetic cream can be expected.

The injection is a preparation which is directly applied into a body intracutaneously or transcutaneously or transmucosally in a form of a pharmaceutical solution, suspension, emulsion, or a medicine used by being dissolved or suspended in a solvent as necessary. The types of the injections include an aqueous injection, a nonaqueous injection, a suspension injection, an emulsion injection, and a solid injection used by dissolution or suspension, depending on the physical properties of the solvent and the medicine itself.

The dosage form for the internal medicine includes a granule, a fine granule, a powder, a coated tablet, a tablet, a pulvis, a (micro) capsule preparation, a chewable preparation, a syrup, a juice, a liquid preparation, a suspension, an emulsion and the like.

The implant preparation is a solid preparation intended to be subcutaneously implanted, and includes the solid injection and other solid preparations. The (micro) capsule preparation or a microneedle preparation described below may be used as an implant preparation.

The percutaneous absorption preparation is a preparation for administering a drug through a skin or a mucosa by local application and pasting, and includes a liquid material, an ointment, a cream preparation, a tape preparation, a patch preparation, a poultice preparation and the like which contain medicinal ingredients.

The microneedle preparation is a preparation in a form of one or a plurality of fine needles, and also includes a microneedle array in which a plurality of microneedles are formed on a surface of a substrate. The shape of the microneedle is exemplified by a spindle shape, a frustum shape or a cone shape formed of a water-soluble or water-swellable resin, and a size of one microneedle is typified by 0.15 to 1.0 mm in one side or a diameter of its base and about 0.1 to 2.0 mm in a height. The water-soluble or water-swellable resin may be any resin capable of dissolving or swelling in vivo, and it includes, for example, a polysaccharide such as glycogen, dextrin, dextran, dextran sulfate, sodium chondroitin sulfate, hydroxypropyl cellulose, chitosan, alginic acid, agarose, chitin, chitosan, pullulan, amylopectin, starch and hyaluronic acid; a protein such as collagen, gelatin and albumin; a synthetic polymer such as polyvinyl alcohol, carboxyvinyl polymer, sodium polyacrylate, polyvinylpyrrolidone and polyethylene glycol, and among them, hyaluronic acid, collagen, gelatin, polyvinylpyrrolidone and polyethylene glycol are preferred. Therefore, the PEG-graft-HA can be used alone as a resin for forming the microneedle, or can also be used in combination with one or more polymeric substances selected from the water-soluble or water-swellable resins.

The cosmetic lotion, cosmetic emulsion or cosmetic cream is a product which can be used as a cosmetic and includes products classified as a quasi drug or a medical cosmetic.

A medicine which is an injection, an internal medicine, an implant preparation, a percutaneous absorption preparation or a microneedle preparation is prepared by formulating them together with active ingredients using a PEG-carboxy group-containing polysaccharide graft as a carrier by a conventional method. Furthermore, various pharmaceutically acceptable substances for preparations can be blended according to the necessity by the preparations. The substance for the preparation can be appropriately selected depending on the dosage form of the preparation, and it includes, for example, an excipient, a diluent, an additive, a disintegrant, a binder, a coating agent, a lubricant, a glidant, a lubricating agent, a flavoring agent, a sweetening agent, a solubilizer, a solvent, a base, an adhesive and the like.

A cosmetic which is a cosmetic lotion, a cosmetic emulsion or a cosmetic cream is prepared by formulating them together with cosmetic raw materials and optionally valuable components using a PEG-carboxy group-containing polysaccharide graft as a carrier by a conventional method. Among the PEG-carboxy group-containing polysaccharide grafts, the PEG-graft-HA can be expected to sustain effects by sustained release, because the hyaluronic acid itself has a moisturizing effect and an anti-wrinkle effect for the skin.

### Effects of Invention

When the proteinaceous medicine is dissolved in an aqueous two-phase system prepared by dissolving PEG and a carboxy group-containing polysaccharide in water, an extremely large amount of proteinaceous medicine is distributed in the PEG phase. However, if the system is dissolved as it is, PEG diffuses in body fluid (blood, intercellular fluid), and thus this aqueous two-phase system was difficult to use as a sustained-release preparation. However, when the PEG-carboxy group-containing polysaccharide graft prepared by grafting PEG into a carboxy group-containing polysaccharide is used, the whole molecular weight is high, the diffusion in body fluid can be suppressed, and the graft can be effectively used as a sustained-release preparation.

The PEG-graft-HA has excellent properties as follows.
1. It has an excellent retention property for a proteinaceous medicine and a low-molecular weight drug and is therefore effective as a sustained-release carrier for drugs.
2. The biodegradability of HA is suppressed and the action as a carrier can be sustained for a long time by grafting PEG.

According to the production method for the PEG-graft-HA of the present invention, a compound represented by the general formula (1) is used as a condensing agent and reacted in a solvent mainly composed of water to obtain the PEG-graft-HA rapidly in high yield.

### Brief Description of Drawings

Figure 1 is a ¹H-NMR chart of the modified hyaluronic acid (PEG-graft-HA-1).
Figure 2 is a gel permeation chromatogram of the modified hyaluronic acid (PEG-graft-HA-1).
Figure 3 shows photographs indicating an anti-wrinkle effect of a microneedle patch of the modified hyaluronic acid (PEG-graft-HA-2).
Figure 4 is a graph indicating enzyme resistances of various modified hyaluronic acids. The horizontal axis represents time (hours).

### Detailed Description

Examples of the present invention will be explained in detail.

In order to produce the PEG-graft-HA, a PEG with an aminated terminal is required. Although it may be produced in a laboratorial manner, a commercial product of methoxypolyethylene glycol having an amino group at the terminal (SUNBRIGHT (MEPA-50H), molecular weight: 5000, manufactured by NOF CORPORATION) may be suitably used.

In order to produce the PEG-graft-HA, a methoxy polyethylene glycol (MEPA-50H) having an amino group at the terminal is added to an HA aqueous solution, which is sufficiently stirred to prepare a mixed aqueous solution. An appropriate amount of DMTMM is added thereto, which is subjected to a condensation reaction for several hours. Low-molecular weight products after the reaction can be removed by dialysis. Alternatively, for purification, the reaction product PEG-graft-HA may be precipitated by adding excess acetone to the reaction system to remove the supernatant. Thereafter, the solution was lyophilized and dried at room temperature to prepare a dry powder. **Example 1** (not according to the invention)

### (Preparation of PEG-graft-HA-1)

100 ml of water was put in a 500 ml flask, to which 0.1 g of hyaluronic acid (FCH-SU, molecular weight: 8 to 110,000, manufactured by Kikkoman Biochemifa Company) was added and dissolved by stirring at room temperature. 0.1 g of methoxypolyethylene glycol having an amino group at the terminal (SUNBRIGHT (MEPA-50H)) was added thereto, and stirred to prepare a uniform solution. Furthermore, 0.1 g of DMTMM was added thereto at room temperature to initiate a condensation reaction, and reacted for 5 hours. After completion of the reaction, purification was carried out by removing the low-molecular weight products by dialysis for 24 hours. Thereafter, the solution was lyophilized to obtain a powder of the PEG-graft-HA-1.

The resulting PEG-graft-HA-1 was dissolved in 0.01 M phosphate buffer (pH 7.5: hereinafter referred to as "PBS") to prepare a 4 WT% aqueous solution. Its transmittance at 650 nm was 28%. The transmittances (at 650 nm) of the raw material hyaluronic acid and the 4 WT% SUNBRIGHT aqueous solution were 95% and 100% respectively. It is considered that this difference is attributed to a microphase-separated structure formed by grafting the PEG chain into the hyaluronic acid, and as a result of which the PEG-graft-HA was confirmed. Note that WT% means % by weight, and the same applies to the following.

### (Characterization of the product after graft reaction of hyaluronic acid)

A test solution was prepared by dissolving the PEG-graft-HA-1 prepared and dried according to Example 1 in a heavy water so that its concentration was 1 WT%. As a nuclear magnetic resonance (NMR) apparatus, JEOL-LA 300FT NMR SYSTEM (manufactured by JEOL Ltd.) was used. For data analysis, JEOL NMR DataProcessing Software ALICE 2 was used.

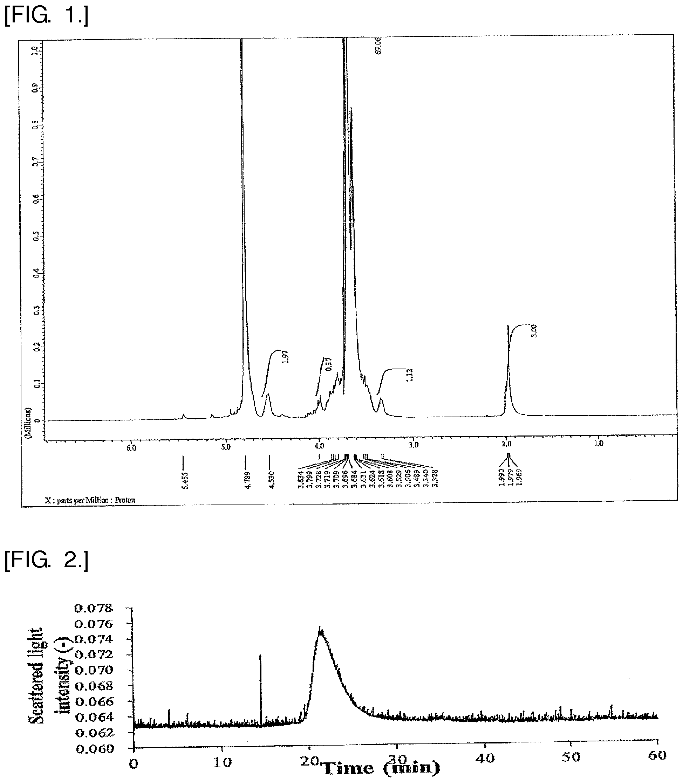

The obtained NMR chart is shown in Figure 1 (¹H-NMR (400 MHz, D₂O). In Figure 1, the peak δ (ppm) = 1.97 is derived from the methyl terminal of HA at NHCO-CH₃ in hyaluronic acid. The peak δ (ppm) = 3.5-3.7 is derived from the ethylene group at -CH₂CH₂O-. From this NMR chart, it can be seen that PEG is grafted into the hyaluronic acid. From the areas of both peaks, the graft rate of PEG into the hyaluronic acid (graft rate based on weight ratio) was calculated to be 41%.

The graft rate was obtained by calculating the ratio between the number of the methyl groups of the HA and the number of hydrogen atoms in the ethylene group of PEG from the NMR measurement result. That is, the graft ratio is a value indicating the weight ratio of the grafted PEG and the HA in percentage.

In chemical shift of ¹H-NMR (TMS standard), the chemical shift of the ethylene group of PEG is at δ (ppm) = about 3.5-3.7. The peak area in the chemical shift specific to the carboxy group-containing polymer (polysaccharide) and the peak area in the chemical shift of the ethylene group of PEG are calculated, and a ratio therebetween is calculated, so that the graft ratio of PEG grafted to the carboxy group-containing polymer (polysaccharide ) can be determined.

### (Gel permeation chromatography (GPC) of PEG-graft-HA-1)

For the graft reactant, the unity confirmation and the molecular weight measurement were performed by size exclusion chromatograph (SEC-MALS) with a multi-angle light scattering detector. An aqueous solution containing 0.1 wt% of the PEG-graft-HA prepared and dried according to Example 1 was used as a sample. The measurement conditions were as follows.
Column: Shodex PROTEIN GF-710 HQ (7.6 mm I.D. x 300 mm) + Shodex Asahipak GS-510 HQ (7.5 mm I.D. x 300 mm)
Injection volume: 150 µL
Eluent: 0.1 M Phosphate buffer (pH 7.0)
Flow rate: 1.0 mL/min
Detection: MALS (Multi angle laser light scattering), 90 degrees
Column temperature: 26°C

The obtained chromatogram is shown in Figure 2. The peak is a single peak, and only PEG-graft-HA is detected. Unreacted PEG and hyaluronic acid were not detected. The molecular weight was calculated to be 443,000.

### Example 2 (not according to the invention)

### (Sustained release of insulin using PEG-graft-HA-2)

A PEG-graft-HA-2 (49.7 mg) synthesized mostly in the same manner as Example 1 except that, instead of the FCH-SU, FCH-80LE (molecular weight: 60 to 1,000,000, manufactured by Kikkoman Biochemifa Company) was used as the HA, and a HA-80LE (28.9 mg) were dissolved in 0.7 ml of PBS to prepare a solution containing 4 wt% of HA. The solution was cloudy.

0.87 mg of FITC-Insulin (insulin labeled with FITC to facilitate fluorescence measurement, made by ourselves) was dissolved in the above-described solution to obtain a yellow and transparent solution. The solution was transferred to a dialysis tube (dialysis membrane with a cutoff molecular weight of 3,000, manufactured by Spectrum Laboratories, Inc.), and the tube was immersed in a test solution (500 ml) in a beaker (500 ml) so that the dialysis tube was fixed so as not to move. The PBS was used as the test liquid. The PBS was previously allowed to stand at room temperature for 1 day or longer. A stirring bar was put in the beaker and rotated at 145 rpm using a magnetic stirrer. The upper part of the experimental system was covered with a plastic wrap so that the test liquid did not evaporate and the liquid volume did not change. 1 mL of test liquid was quickly sampled from the vicinity of the center of the beaker every 10 hours. Immediately after every sampling, 1 mL of PBS was gently added, and the liquid volume of the test liquid was kept constant at all times. Fluorescence of the sampled test liquid was measured at an excitation wavelength of 495 nm, a measurement wavelength of 510 to 600 nm and a temperature of 25°C. The amount of FITC-Insulin was determined based on the fluorescence intensity at 520 nm to calculate a release rate. The results are shown as Example 2 in Table 1.

The same FITC-Insulin release experiment was carried out using the HA instead of the PEG-graft-HA-2. The results are shown as Comparative Example 1 in Table 1.

**[Table 1]**

| | release rate of insulin (%) | | | |
|---|---|---|---|---|
| time (h) | **10** | **20** | **40** | **60** |
| Example 2 | 6 | 7 | 8 | **10** |
| Comparative Example 1 | **55** | **70** | **76** | **81** |

The insulin accumulated in the PEG-graft-HA-2 is extremely slowly released to the outside compared to the insulin accumulated in the inside of the hyaluronic acid. Thus, the PEG-graft-HA-2 has a strong action of sustained release for insulin and is a carrier suitable for the sustained-release DDS.

### Example 3 (not according to the invention)

### (Confirmation of suppressed enzymatic degradability using PEG-graft-HA-2)

The PEG-graft-HA-2 synthesized in Example 2 was dissolved in 0.15 mole% PBS so that its concentration was 2 WT%. Similarly the HA was also dissolved in 0.15 mole% PBS so that its concentration of the HA was 2 WT%. Hyaluronidase (Hyaluronidase "Amano", manufactured by Wako Pure Chemical Industries, Ltd.) was added to each solution so that its concentration was 30 units/mg HA, and decomposition of the hyaluronic acid was measured by measuring the decrease in the molecular weight of the hyaluronic acid.

The decreasing rate of the molecular weight 8 hours after the addition of the hyaluronidase (molecular weight after 8 hours/initial molecular weight) was 70% in the case of the PEG-graft-HA-2, and 9% in the case of the hyaluronic acid. In the single system of the hyaluronic acid, the molecular weight decreased to one tenth or less after 8 hours, but in the PEG-graft-HA-2, the decreasing rate of the molecular weight was 70%, and it was revealed that the enzymatic degradation of the hyaluronic acid was considerably suppressed by grafting PEG.

### Example 4 (not according to the invention)

### (Use for microneedle)

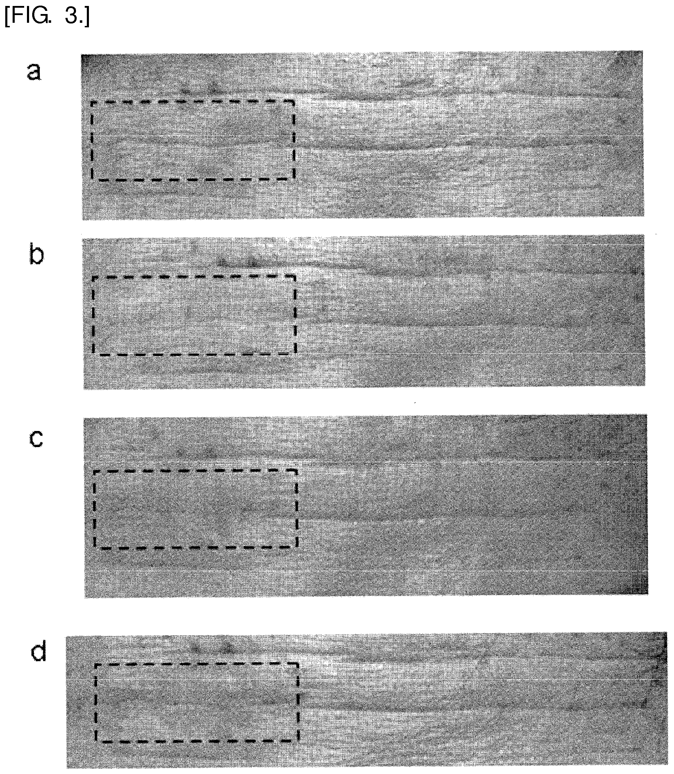

A microneedle was prepared using the PEG-graft-HA-2, which was applied to wrinkle parts on foreheads of volunteers in our company, and sustainability of an anti-wrinkle effect was verified and tested. A medical microneedle (needle length: 800 µm) was used to make it easy to observe the effect with the naked eye. The microneedle was formed by a mold method to prepare an elliptical microneedle patch with a long diameter of 1.0 cm and a short diameter of 0.6 cm, which was attached to a wrinkle part on a forehead, and its time-dependent change was observed to obtain the results in Figure 3.

The microneedle was applied along the deep wrinkle surrounded by the dashed line in Figure 3, and after 5 hours, it was peeled off. Herein, (a) shows a state before use, (b) shows a state after 3 days, (c) shows a state after 7 days, and (d) shows a state after 12 days.

It was applied once a day continuously for 3 days, and applied once again in the next week to observe the time-dependent change of the wrinkle at the application part. As shown in Figure 3, an effect of smoothing the wrinkle was clearly observed by the continuous application for 3 days. In addition, although the effect slightly decreases with time, it can be seen that the effect is sustained during the two-week observation compared to before the use. In past studies, wrinkles gradually returned 7 days after termination of application by any usual hyaluronic acid patch, meanwhile the PEG-graft-HA slowly metabolized and was proved to have a possibility as a cosmetic with a sustained anti-wrinkle effect.

### Example 5

### (Production of hyaluronic acid-PEG graft substance by esterification)

All reagents were purchased from Wako Pure Chemical Industries, Ltd., and special-grade reagents were used. 6.0 g of hyaluronic acid (FCH-SU, molecular weight: 8 to 110,000, manufactured by Kikkoman Biochemifa Company) was suspended in 100 g of THF and 50 g of PEG400, to which 1 ml of concentrated sulfuric acid was added, and reacted while stirring at 50°C for 10 hours. After 10 hours, the reactant was taken out, purified and dried (referred to as PEG-HA-5).

### Example 6

### (Production of hyaluronic acid-PEG graft substance by esterification - 2)

6.0 g of hyaluronic acid (FCH-SU) was suspended in 70 g of PEG400, to which 1 ml of perchloric acid was added, and reacted while stirring at 50°C for 10 hours. After 20 hours, the reactant was taken out, purified and dried (referred to as PEG-HA-6).

### Example 7

### (Measurement of graft rate by infrared spectrum)

The PEG graft rates in the PEG-HA-5 and the PEG-HA-6 were quantified by infrared spectroscopy. Focusing on a peak of PEG at 2870 cm⁻¹ and a peak of the hyaluronic acid at 1025 cm⁻¹, infrared spectra of various blends of PEG and the hyaluronic acid were measured (intensities at 2870 cm⁻¹/1025 cm⁻¹) to prepare a calibration curve configured by composition ratios of both polymers. Subsequently, the infrared spectra of the PEG-HA-5 and the PEG-HA-6 were measured to calculate the graft rates from the calibration curve. For the infrared spectrometer, a high-speed FT-IR imaging system (model: Spectrum 100 FT-IR/Spotlight 400, manufactured by PerkinElmer Japan Co., Ltd.) was used. The calculated PEG graft rates were 0.27 in the PEG-HA-5 and 0.29 in the PEG-HA-6.

### Example 8

### (Confirmation of suppressed enzymatic degradability using PEG-graft-HA)

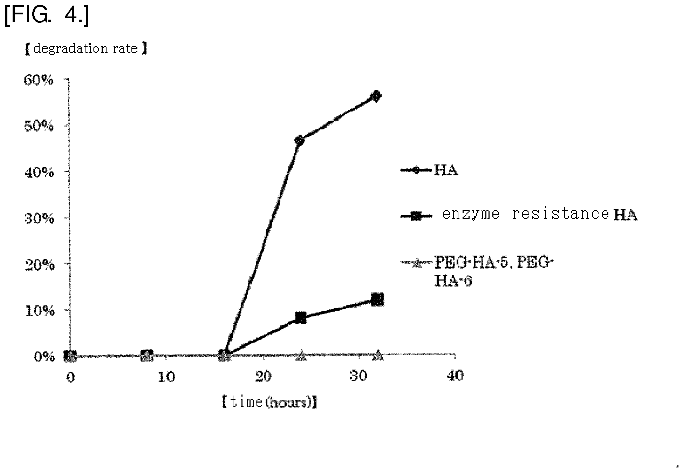

An enzymatic decomposition suppression test was carried out using the PEG-HA-5, the PEG-HA-6, a hyaluronic acid to which enzyme resistance is imparted manufactured by Q company, and an HA (FCH-SU 9) for reference. Each sample was dissolved in 0.15 mole% PBS so that the sample concentration was 2 WT%. A hyaluronidase (Hyaluronidase "Amano", manufactured by Wako Pure Chemical Industries, Ltd.) was added to each solution so that its concentration was 2 units/mg HA, and degradation rates were calculated from the decreased molecular weights after 0, 8, 16, 24 and 32 hours and plotted to prepare Figure 4. Decomposition of hyaluronic acid was measured by measuring the decreased molecular weight of the hyaluronic acid.

The PEG-HA-5 and the PEG-HA-6 showed far higher enzyme resistances compared to not only the hyaluronic acid but also the commercial modified hyaluronic acid appealing enzyme resistance.

## Claims

1. A drug delivery system carrier composed of a PEG-carboxy group-containing polysaccharide graft, wherein a carboxy group and PEG are grafted through an ester bond, wherein
the carboxy group-containing polysaccharide is hyaluronic acid, and wherein a weight ratio of PEG and hyaluronic acid is 1 : 20 to 1 : 0.1.

2. An injection containing the drug delivery system carrier according to claim 1.

3. An internal medicine containing the drug delivery system carrier according to claim 1.

4. An implant preparation containing the drug delivery system carrier according to claim 1.

5. A percutaneously absorbable preparation containing the drug delivery system carrier according to claim 1.

6. A cosmetic containing the drug delivery system carrier according to claim 1.

7. A microneedle preparation containing the drug delivery system carrier according to claim 1.

8. A sustained release insulin composed of an insulin and a PEG-carboxy group-containing polysaccharide graft, wherein a carboxy group and PEG are grafted through an ester bond,
wherein the carboxy group-containing polysaccharide is hyaluronic acid, and wherein a weight ratio of PEG and hyaluronic acid is 1 : 20 to 1 : 0.1.

9. A sustained release exendin-4 composed of an exendin-4 and a PEG-carboxy group-containing polysaccharide graft, wherein a carboxy group and PEG are grafted through an ester bond.
wherein the carboxy group-containing polysaccharide is hyaluronic acid, and wherein a weight ratio of PEG and hyaluronic acid is 1 : 20 to 1 : 0.1.

10. A production method for a PEG-carboxy group-containing polysaccharide graft, wherein the carboxy group-containing polysaccharide is hyaluronic acid, the method comprising a step of condensing PEG and hyaluronic acid in a nonaqueous state in coexistence with an acid, wherein PEG and a carboxy group of the polysaccharide are grafted through an ester bond,
wherein a weight ratio of PEG and hyaluronic acid is 1 : 20 to 1 : 0.1.

## Patentansprüche

1. Träger für ein Arzneimittelabgabesystem, bestehend aus einem PEG-Carboxygruppen-enthaltenden Polysaccharidpfropf, wobei eine Carboxygruppe und PEG durch eine Esterbindung gepfropft sind, wobei
das Carboxygruppen-enthaltende Polysaccharid Hyaluronsäure ist, und wobei das Gewichtsverhältnis von PEG und Hyaluronsäure 1:20 bis 1:0,1 beträgt.

2. Injektion, enthaltend den Träger für ein Arzneimittelabgabesystem nach Anspruch 1.

3. Inneres Arzneimittel, enthaltend den Träger für ein Arzneimittelabgabesystem nach Anspruch 1.

4. Implantatpräparat, enthaltend den Träger für ein Arzneimittelabgabesystem nach Anspruch 1.

5. Perkutan resorbierbares Präparat, enthaltend den Träger für ein Arzneimittelabgabesystem nach Anspruch 1.

6. Kosmetikum, enthaltend den Träger für ein Arzneimittelabgabesystem nach Anspruch 1.

7. Mikronadelpräparat, enthaltend den Träger für ein Arzneimittelabgabesystem nach Anspruch 1.

8. Insulin mit verzögerter Freisetzung, bestehend aus einem Insulin und einem PEG-Carboxygruppen-enthaltenden Polysaccharidpfropf, wobei eine Carboxygruppe und PEG durch eine Esterbindung gepfropft sind,
wobei das Carboxygruppen-enthaltende Polysaccharid Hyaluronsäure ist, und wobei das Gewichtsverhältnis von PEG und Hyaluronsäure 1:20 bis 1:0,1 beträgt.

9. Exendin-4 mit verzögerter Freisetzung, bestehend aus einem Exendin-4 und einem PEG-Carboxygruppen-enthaltenden Polysaccharidpfropf, wobei eine Carboxygruppe und PEG durch eine Esterbindung gepfropft sind,
wobei das Carboxygruppen-enthaltende Polysaccharid Hyaluronsäure ist, und wobei das Gewichtsverhältnis von PEG und Hyaluronsäure 1:20 bis 1:0,1 beträgt.

10. Verfahren zur Herstellung eines PEG-Carboxygruppen-enthaltenden Polysaccharidpfropfs, wobei das Carboxygruppen-enthaltende Polysaccharid Hyaluronsäure ist, wobei das Verfahren einen Schritt des Kondensierens von PEG und Hyaluronsäure in einem nicht-wässrigen Zustand in gleichzeitiger Gegenwart einer Säure umfasst, wobei PEG und eine Carboxygruppe des Polysaccharids durch eine Esterbindung gepfropft werden,
wobei das Gewichtsverhältnis von PEG und Hyaluronsäure 1:20 bis 1:0,1 beträgt.

## Revendications

1. Vecteur de système d'administration de médicament composé d'un greffon de polysaccharide contenant du PEG et un groupe carboxy, dans lequel un groupe carboxy et du PEG sont greffés par une liaison ester, dans lequel
le polysaccharide contenant un groupe carboxy est l'acide hyaluronique, et dans lequel un rapport pondéral du PEG et de l'acide hyaluronique est de 1:20 à 1:0,1.

2. Injection contenant le vecteur de système d'administration de médicament selon la revendication 1.

3. Médicament à usage interne contenant le vecteur de système d'administration de médicament selon la revendication 1.

4. Préparation d'implant contenant le vecteur de système d'administration de médicament selon la revendication 1.

5. Préparation absorbable par voie percutanée contenant le vecteur de système d'administration de médicament selon la revendication 1.

6. Cosmétique contenant le vecteur de système d'administration de médicament selon la revendication 1.

7. Préparation pour micro-aiguille contenant le vecteur de système d'administration de médicament selon la revendication 1.

8. Insuline à libération prolongée composée d'une insuline et d'un greffon de polysaccharide contenant du PEG et un groupe carboxy, dans laquelle un groupe carboxy et du PEG sont greffés par une liaison ester,
dans laquelle le polysaccharide contenant un groupe carboxy est l'acide hyaluronique, et dans laquelle un rapport pondéral du PEG et de l'acide hyaluronique est de 1:20 à 1:0,1.

9. Exendine-4 à libération prolongée composée d'une exendine-4 et d'un greffon de polysaccharide contenant du PEG et un groupe carboxy, dans laquelle un groupe carboxy et du PEG sont greffés par une liaison ester,
dans laquelle le polysaccharide contenant un groupe carboxy est l'acide hyaluronique, et dans laquelle un rapport pondéral du PEG et de l'acide hyaluronique est de 1:20 à 1:0,1.

10. Procédé de production d'un greffon de polysaccharide contenant du PEG et un groupe carboxy, dans lequel le polysaccharide contenant un groupe carboxy est l'acide hyaluronique, le procédé comportant une étape consistant à condenser du PEG et de l'acide hyaluronique dans un état non aqueux en coexistence avec un acide, dans lequel le PEG et un groupe carboxy du polysaccharide sont greffés par une liaison ester,
dans lequel un rapport pondéral du PEG et de l'acide hyaluronique est de 1:20 à 1:0,1.
